# EUROPÄISCHE PATENTSCHRIFT

(11) **EP 0 852 137 B1**
(45) Veröffentlichungstag und Bekanntmachung des Hinweises auf die Patenterteilung: **10.08.2005**
(21) Anmeldenummer: 97119397.4
(22) Anmeldetag: 06.11.1997
(51) Int. Cl.: A61K 7/42, C07C 225/14, C07C 229/30, C07C 255/30, A61K 31/13, A61K 31/22

(54) **Photostabile UV-A-Filter enthaltende kosmetische Zubereitungen**
Cosmetic compositions containing a photostable UV-A filter
Compositions cosmétiques contenant un filtre UV-A photostable

(30) Priorität: 29.11.1996 DE 19649381; 21.03.1997 DE 19712033
(43) Veröffentlichungstag der Anmeldung: 08.07.1998
(73) Patentinhaber: BASF AKTIENGESELLSCHAFT, 67056 Ludwigshafen (DE)
(72) Erfinder: Habeck, Thorsten Dr., 67149 Meckenheim (DE); Aumüller, Alexander Dr., 67435 Neustadt (DE); Schehlmann, Volker Dr., 67354 Römerberg (DE); Westenfelder, Horst, 67435 Neustadt (DE); Wünsch, Thomas Dr., 67346 Speyer (DE)

(56) Entgegenhaltungen:
- DE-A- 3 825 382
- FR-A- 2 285 881
- US-A- 3 079 366
- US-A- 4 514 383
- F. B. DAINS ET AL.: "On the reactions of the formamidine derivatives" JOURNAL OF THE AMERICAN CHEMICAL SOCIETY., Bd. 31, 1909, Seiten 1148-1157, XP000864589 DC US
- F. B. DAINS ET AL.: "On the reactions of the formamidines." JOURNAL OF THE AMERICAN CHEMICAL SOCIETY., Bd. 40, 1918, Seiten 562-569, XP000864587 DC US
- R. K. MAPARA ET AL.: "Studies in the synthesis of 4-hydroxyquinolines" JOURNAL OF THE INDIAN CHEMICAL SOCIETY, Bd. 31, Nr. 12, 1954, Seiten 951-956, XP000864596
- M. MICHALIK: "NMR-Spektroskopische Untersuchungen an 2-Acetyl-3-amino-acrylsäureestern" JOURNAL FUER PRAKTISCHE CHEMIE, Bd. 327, Nr. 6, 1985, Seiten 908-916, XP000864588 LEIPZIG, DE ISSN: 0021-8383
- OTTO S. WOLFBEIS: "Substituierte Chinoline aus 2-Arylaminomethylen-1,3-diketonen" SYNTHESIS,Oktober 1977 (1977-10), Seiten 723-725, XP000907015
- DATABASE WPI Section Ch, Week 198712 Derwent Publications Ltd., London, GB; Class A60, AN 1987-084265 XP002138150 & JP 62 036484 A (AJINOMOTO KK), 17. Februar 1987 (1987-02-17)

## Beschreibung

Die Erfindung betrifft die Verwendung von Enaminderivaten als photostabile UV-Filter in kosmetischen und pharmazeutischen Zubereitungen zum Schutz der menschlichen Epidermis oder menschliche Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschuztmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PAR-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP 0514491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP 251 398 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

DE 3825382 A1 beschreibt N-substituierte 2-Aminovinylverbindungen und deren Verwendung als UV-B-Filter in kosmetischen Zubereitungen.

US 3,079,366 beschreibt substituierte Arylaminoethylenverbindungen als UV-Stabilisatoren für Kunststoffe.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweise und je nach Substituent in Öl oder Wasser löslich sind.

Diese Aufgabe wurde erfindungsgemäß gelöst durch Verwendung von Verbindungen der Formel I 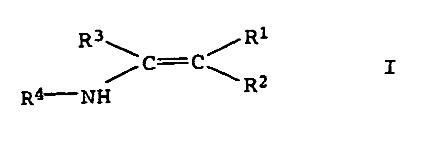 in der die C=C Doppelbindung in der E oder Z Konfiguration vorliegt die Variablen folgende Bedeutung haben:
- R¹: COOR⁵, COR⁵, CN;
- R²: COOR⁶, COR⁶, CN;
- R³: Wasserstoff;
- R⁴: Phenyl, substituiert mit C₁-C₁₂-Alkoxyresten;
- R⁵ und R⁶: unabhängig voneinander offenkettige oder verzweigte aliphatische, oder gegebenenfalls substituierte aromatische Reste mit jeweils bis zu 8 C-Atomen,
als UV-A-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Substituenten kommen sowohl lipophile als auch hydrophile Substituenten mit z.B. bis zu 20 C-Atomen in Betracht. Lipophile d.h. die Öllöslichkeit der Verbindungen der Formel I verstärkende Reste sind z.B. aliphatische oder cycloaliphatische Reste insbesondere Alkylreste mit 1 bis- 18 C-Atomen, Alkoxy-, Mono- und Dialkylamino-, Alkoxycarbonyl-, Mono- und Dialkylaminocarbonyl-, Mono- und Dialkylaminosulfonylreste, ferner Cyan-, Nitro-, Brom-, Chlor-, Iod- oder Fluorsubstituenten.

Als Alkoxyreste kommen solche mit 1 bis 12 C-Atomen, vorzugsweise mit 1 bis 8 C-Atomen in Betracht.

Beispielsweise sind zu nennen:

| | |
|---|---|
| methoxy | isopropoxy- |
| n-propoxy- | 1-methylpropoxy- |
| n-butoxy- | n-pentoxy- |
| 2-methylpropoxy- | 3-methylbutoxy- |
| 1,1-dimethylpropoxy- | 2,2-dimethylpropoxy- |
| hexoxy- | 1-methyl-1-ethylpropoxy- |
| heptoxy- | octoxy- |
| 2-ethylhexoxy- | |

Alkoxycarbonylreste sind z.B Ester, die die oben genannten Alkoxyreste oder Reste von höheren Alkoholen z.B. mit bis zu 20 C-Atomen, wie iso-C₁₅-Alkohol, enthalten.

Die erfindungsgemäß zu verwendenden Verbindungen der Formel I können nach der Gleichung 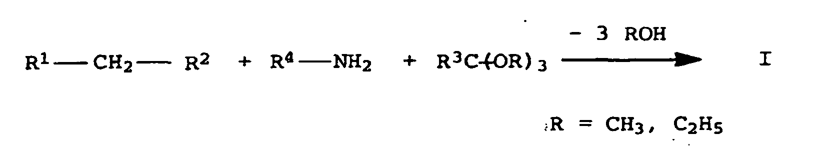 durch Kondensation hergestellt werden, wobei R¹ bis R⁴ die oben genannte Bedeutung haben.

Beispielsweise ergibt die Umsetzung von 2,4-Pentandion mit Anthranilsäure-2-ethylhexylester und Triethylorthoformiat die Verbindung 24 in Tab. 2.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkoimmission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-B-Filtersubstanzen, die in Kombination mit den erfindungsgemäß zu verwendenden Verbindungen der Formel I angewandt werden, kommen beliebige UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 7/6/7-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-(4'-Methyl)benzyliden-bornan-2-on | 36861-47-9 |
| 15 | 3-Benzylidenbornan-2-on | 16087-24-8 |
| 16 | 1-(4'-Isopropylphenyl)-3-phenylpropan-1,3-dion | 63260-25-9 |
| 17 | 4-Isopropylbenzylsalicylat | 94134-93-7 |
| 18 | 2,4,6-Trianilin-(o-carbo-2'-ethylhexyl-1'-oxy)-1,3,5-triazin | 88122-99-0 |
| 19 | 3-Imidazol-4-yl-acrylsäure und ihr Ethylester | 104-98-3* |
| 20 | 2-Cyano-3,3-diphenylacrylsäureethylester | 5232-99-5 |
| 21 | 2-Cyano-3,3-diphenylacrylsäure-2'-ethylhexylester | 6197-30-4 |
| 22 | Menthyl-o-aminobenzoate oder: 5-Methyl-2-(1-methylethyl)-2-aminobenzoate | 134-09-8 |
| 23 | Glyceryl p-aminobenzoat oder: 4-Aminobenzoesäure-1-glyceryl-ester | 136-44-7 |
| 24 | 2,2'-Dihydroxy-4-methoxybenzophenon (Dioxybenzone) | 131-53-3 |
| 25 | 2-Hydroxy-4-methoxy-4-methylbenzophenon (Mexonon) | 1641-17-4 |
| 26 | Triethanolamin Salicylat | 2174-16-5 |
| 27 | Dimethoxyphenylglyoxalsäure oder: 3,4-dimethoxy-phenyl-glyoxal-saures Natrium | |
| 28 | 3-(4'Sulfo)benzyliden-bornan-2-on und seine Salze | 56039-58-8 |

Schließlich sind auch mikronisierte Pigmente wie Titandioxid und Zinkoxid zu nennen.

Zum Schutz menschlicher Haare vor UV-Strahlen können die erfindugsgemäßen Lichtschutzmittel der Formel I in Shampoos, Lotionen, Gelen oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Gegenstand der Erfindung sind auch die Verbindungen der Formel I zur Verwendung als Medikament sowie pharmazeutische Mittel zur vorbeugenden Behandlung von Entzündungen und Allergien der Haut sowie zur Verhütung bestimmter Hautkrebsarten, welche eine wirksame Menge mindestens einer Verbindung der Formel I als Wirkstoff enthalten.

### Beispiele:

### I. Herstellung

### Beispiel 1

### Allgemeine Vorschrift (für die Verbindung der Nr. 1 der Tabelle 2)

0,1 mol p-Aminobenzoesäure-2-ethylhexylester, 0,1 mol Pivaloylacetonitril und 0,1 mol Triethylorthoformiat wurden in 100 ml Diethylenglykol 2 h auf 120°C erhitzt, wobei Ethanol abdestilliert wurde. Nach Abkühlung auf 80°C wurde mit Wasser versetzt und vom ausgefallenen Niederschlag abfiltriert. Anschließend wurde aus Petrolether umkristallisiert. Man erhielt in 80 %iger Ausbeute Verbindung 1 der Tab. 2.

### Beispiel 2

0,1 mol Anthranilsäure-2-ethylhexylester, 0,1 mol 2,4-Pentandion und 0,1 mol Triethylorthoformiat wurden in 100 ml Diethylenglykol 2 h auf 120°C erhitzt, wobei Ethanol abdestilliert wurde. Nach Abkühlung auf 80°C wurde mit Wasser versetzt und vom ausgefallenen Niederschlag abfiltriert. Anschließend wurde aus Petrolether umkristallisiert. Man erhielt in 70 %iger Ausbeute Verbindung 24 der Tab. 2.

### Beispiel 3

0,1 mol m-Toluidin, 0,1 mol Pivaloylacetonitril und 0,1 mol Triethylorthoformiat und 1 g Zinkchlorid wurden in 100 ml Diethylenglykol 2 h auf 120°C erhitzt, wobei Ethanol abdestilliert wurde. Nach Abkühlung auf 80°C wurde mit Wasser versetzt und vom ausgefallenen Niederschlag abfiltriert. Anschließend wurde aus Petrolether umkristallisiert. Man erhielt in 70 %iger Ausbeute Verbindung 2 der Tab. 2.

Weitere so hergestellte Verbindungen sind in Tabelle 2 angegeben.

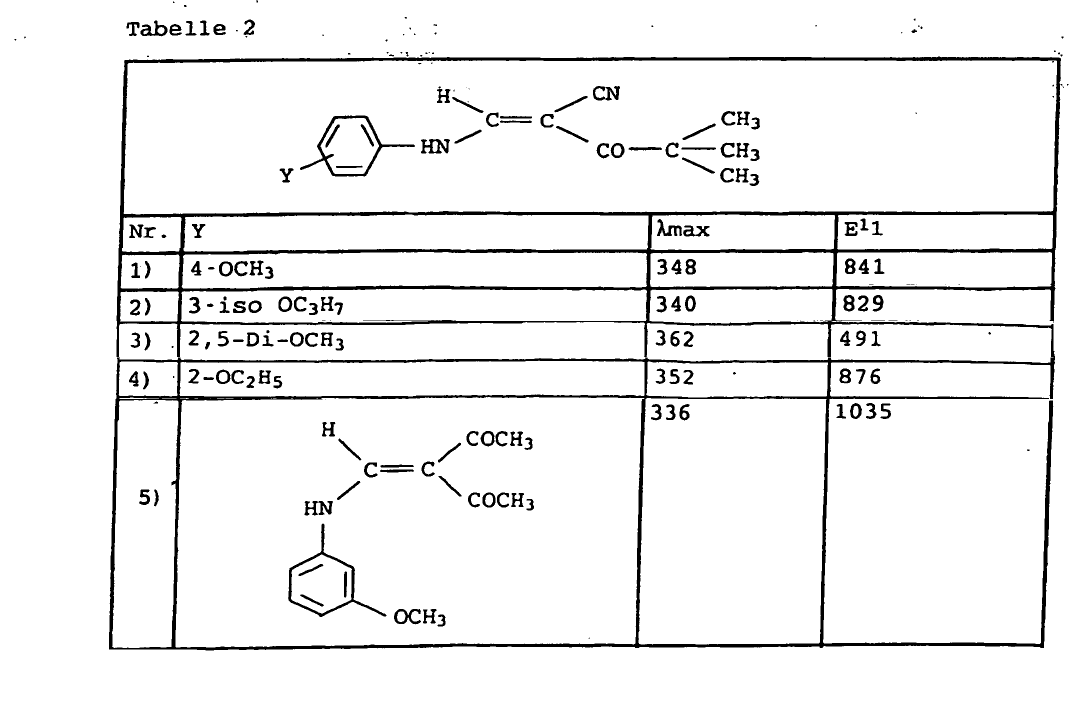

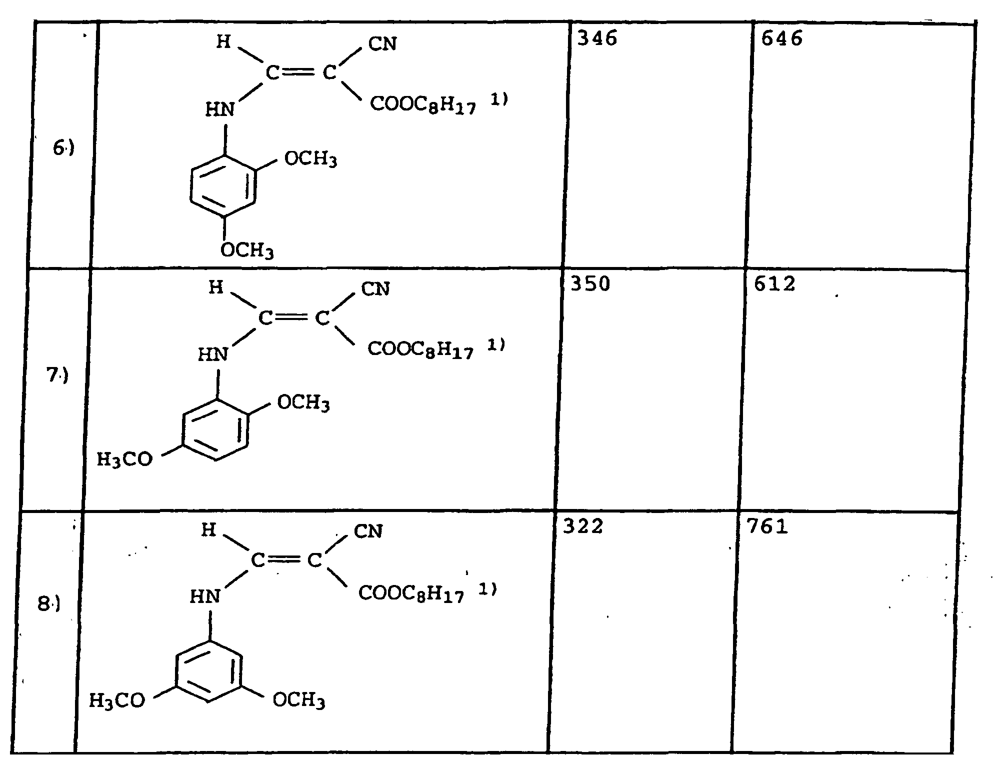

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

## Patentansprüche

1. Verwendung von Verbindungen der Formel I 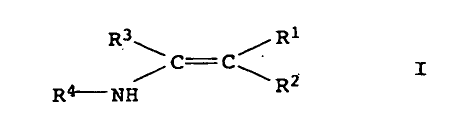 in der die C=C Doppelbindung in der E oder Z Konfiguration vorliegt und die Variablen folgende Bedeutung haben:
R¹ CN, COOR⁵, COR⁵;
R² CN, COOR⁶, COR⁶;
R³ Wasserstoff;
R⁴ Phenyl, substituiert mit C₁- bis C₁₂-Alkoxyresten;
R⁵ und R⁶ unabhängig voneinander offenkettige oder verzweigte aliphatische oder gegebenenfalls substituierte, aromatische Reste mit jeweils bis zu 8 C-Atomen,
als UV-A-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Kosmetische und pharmazeutische Lichtschutzmittelzubereitungen ausgewählt aus der Gruppe, bestehend aus Ölen, Öl-in-Wasser-Emulsionen, Wasser-in-Öl-Emulsionen, Cremes, Lippenschutzstiftmassen und fettfreien Gelen, zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten im UV-Bereich absorbierenden Verbindungen, als photostabile UV-A-Filter wirksame Mengen von Verbindungen der Formel I 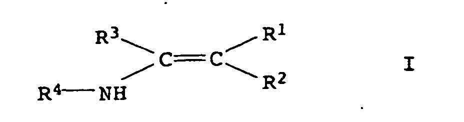 enthalten, in der die Variablen die Bedeutung gemäß Anspruch 1 haben.

## Claims

1. The use of compounds of the formula I 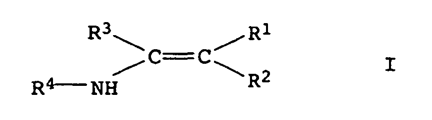 where the C=C double bond has the E or Z configuration, and the variables have the following meanings:
R¹ CN, COOR⁵, COR⁵;
R² CN, COOR⁶, COR⁶;
R³ hydrogen;
R⁴ phenyl substituted by C₁- to C₁₂-alkoxy radicals;
R⁵ and R⁶ independently of one another open-chain or branched aliphatic or unsubstituted or substituted aromatic radicals having up to 8 carbon atoms in each case,
as UV-A filters in cosmetic and pharmaceutical compositions for protecting the human skin or human hair from the sun's rays, alone or together with compounds which are known per se for cosmetic and pharmaceutical compositions and absorb in the UV region.

2. A cosmetic or pharmaceutical sunscreen composition selected from the group consisting of oils, oil-in-water emulsions, water-in-oil emulsions, creams, protective lipstick compositions and nongreasy gels for protecting the human epidermis or human hair from UV light in the range from 280 to 400 nm, which comprises effective amounts of compounds of the formula I 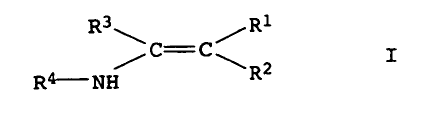 where the variables have the meanings specified in claim 1, as photostable UV-A filters in a cosmetically and pharmaceutically suitable carrier, alone or together with compounds which are known per se for cosmetic and pharmaceutical compositions and absorb in the UV region.

## Revendications

1. Utilisation de composés de la formule I : 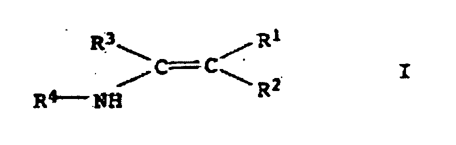 dans laquelle la double liaison C=C se présente dans la configuration E ou Z et les variables ont la signification suivante :
R¹ CN, COOR⁵, COR⁵,
R² CN, COOR⁶, COR⁶,
R³ de l'hydrogène,
R⁴ du phényle, substitué par des radicaux alcoxy en C₁-C₁₂,
R⁵ et R⁶ indépendamment l'un de l'autre des radicaux aliphatiques à chaîne ouverte ou ramifiée ou des radicaux aromatiques, éventuellement substitués, comportant chacun jusqu'à 8 atomes de C,
comme filtre U.V.-A dans des compositions cosmétiques et pharmaceutiques destinées à la protection de la peau humaine ou des cheveux humains vis-à-vis des rayons solaires, seuls ou conjointement avec des composés absorbant dans la gamme U.V. connus en soi pour des compositions cosmétiques et pharmaceutiques.

2. Compositions de produits de protection contre la lumière cosmétiques et pharmaceutiques choisies parmi le groupe constitué des huiles, des émulsions huile-dans-l'eau, des émulsions eau-dans-l'huile, des crèmes, des masses de bâton pour la protection des lèvres et des gels sans graisse, pour la protection de l'épiderme humain ou des cheveux humains contre la lumière U.V. dans la gamme de 280 à 400 nm, **caractérisées en ce qu'**elles contiennent, dans un support approprié du point de vue cosmétique et pharmaceutique, seules ou conjointement avec des composés absorbant dans la gamme U.V. connus en soi pour des compositions cosmétiques et pharmaceutiques, des quantités efficaces de composés de la formule I : 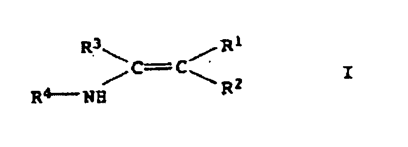 dans laquelle les variables ont la signification selon la revendication 1,
comme filtre U.V.-A photostable.
